# EUROPEAN PATENT APPLICATION

(11) **EP 2 078 751 A1**
(43) Date of publication of application: **15.07.2009**
(21) Application number: 08290022.6
(22) Date of filing: 11.01.2008
(51) Int. Cl.: C12N 15/11, C12Q 1/68

(54) **Specific aptamers generated for suppression of cell migration and invasion by cancer cells, methods of selection for such aptamers and their usage**

(71) Applicant: COMMISSARIAT A L'ENERGIE ATOMIQUE, 75015 Paris (FR)
(72) Inventor: Tavitian, Bertrand, 75015 Paris (FR); Zueva, Elina, 92340 Bourg-La-Reine (FR)
(74) Representative: Thomas, Dean

(57) **Abstract**

The invention relates to a new method to generate aptamers selected using live metastatic cancer cell lines. The Inventors have shown that using their modified SELEX system it is possible to generate metastatic cell specific aptamer molecules and that some of the aptamer molecules they have developed prevent metastatic cell migration.

SEQUENCE LISTING

<110> COMMISSARIAT A L'ENERGIE ATOMIQUE

<120> Specific aptamers generated for suppression of cell migration and
invasion by cancer cells, methods of selection for such aptamers and
their usage

<130> F263-238EP

<160> 38

<170> PatentIn version 3.3

<210> 1
<211> 18
<212> DNA
<213> Artificial

<220>
<223> 1st primer sequence

<400> 1
agattgcact tactcgaa 18

<210> 2
<211> 24
<212> DNA
<213> Artificial

<220>
<223> 2 nd primer sequence

<400> 2
ggaatgaata agctggtatc tccc 24

<210> 3
<211> 81
<212> DNA
<213> Artificial

<220>
<223> Aptamer

<400> 3
gggagatacc agctattcat tccagtgacg acgtcgcccg gttgtctagc ccgcgattca 60

cggttcgagt aagtgcaatc t 81

<210> 4
<211> 81
<212> DNA
<213> Artificial

<220>
<223> aptamer

<400> 4
gggagatacc agctattcat tccgcaacca agtttacgga aataccggct atacgcccga 60

cggttcgagt aagtgcaatc t 81

<210> 5
<211> 82

<212>  DNA
<213>  Artificial

<220>
<223>  aptamer

<400>  5
gggagatacc agctattcat tcccgcgtgt caaacgccgg gtacctccct ttctgcgttg      60

cgggttcgag taagtgcaat ct                                               82


<210>  6
<211>  81
<212>  DNA
<213>  Artificial

<220>
<223>  aptamer

<400>  6
gggagatacc agctattcat tcctacgcca taggacgagg aataggtcct gaaatcttgg      60

gccttcgagt aagtgcaatc t                                                81


<210>  7
<211>  81
<212>  DNA
<213>  Artificial

<220>
<223>  aptamer

<400>  7
gggagatacc agctattcat tccctggcgg cgaaggaaca tccccactcg ctgcatgaca      60

cggttcgagt aagtgcaatc t                                                81


<210>  8
<211>  83
<212>  DNA
<213>  Artificial

<220>
<223>  aptamer

<400>  8
gggagatacc agctattcat tccctggcgg cgaaggaaca tccccactcg ctgcatgaca      60

cacggttcga gtaagtgcaa tct                                              83


<210>  9
<211>  81
<212>  DNA
<213>  Artificial

<220>
<223>  aptamer

<400>  9
gggagatacc agctattcat tccctggcag cgaaggaaca tccccactcg ctgcatgaca      60

cggttcgagt aagtgcaatc t                                                81

<210> 10
<211> 80
<212> DNA
<213> Artificial

<220>
<223> aptamer

<400> 10
gggagatacc agctattcat tccggtacca aggctacctg aatacccgac ggattttccg 60

ggttcgagta agtgcaatct 80


<210> 11
<211> 79
<212> DNA
<213> Artificial

<220>
<223> aptamer

<400> 11
gggagatacc agctattcat tccagccgct gtaatgcgtt catcccattg ccccgcgctg 60

gttcgagtaa gtgcaatct 79


<210> 12
<211> 81
<212> DNA
<213> Artificial

<220>
<223> aptamer

<400> 12
gggagatacc agctattcat tccaagcaca ttcgcttcgg aacttcagcc tgactcttgg 60

ctgttcgagt aagtgcaatc t 81


<210> 13
<211> 81
<212> DNA
<213> Artificial

<220>
<223> aptamer

<400> 13
gggagatacc agctattcat tccccggcac actggtatcc ttacacgtcc ccattccccc 60

tggttcgagt aagtgcaatc t 81


<210> 14
<211> 81
<212> DNA
<213> Artificial

<220>
<223> aptamer

<400> 14
gggagatacc agctattcat tcctagagcc ctgcactagt gcacgtccag ctgtagcagc 60

gggttcgagt aagtgcaatc t 81

<210> 15
<211> 81
<212> DNA
<213> Artificial

<220>
<223> aptamer

<400> 15
gggagatacc agctattcat tcctgatgtg cagacgctcg tggcttaggc catcacatcc 60

gggttcgagt aagtgcaatc t 81


<210> 16
<211> 81
<212> DNA
<213> Artificial

<220>
<223> aptamer

<400> 16
gggagatacc agctattcat tcccaccttc ctggtatcct tactcgtaac agcccgtctc 60

ccgttcgagt aagtgcaatc t 81


<210> 17
<211> 82
<212> DNA
<213> Artificial

<220>
<223> aptamer

<400> 17
gggagatacc agctattcat tccggcgaca cctgccagct cccgacatcg tgtgaaaact 60

gcggttcgag taagtgcaat ct 82


<210> 18
<211> 81
<212> DNA
<213> Artificial

<220>
<223> aptamer

<400> 18
gggagatacc agctattcat tcctagtgcc ccatggccag cccccgagcc atgcgtcctg 60

tgcttcgagt aagtgcaatc t 81


<210> 19
<211> 83
<212> DNA
<213> Artificial

<220>
<223> aptamer

<400> 19
gggagatacc agctattcat tccctggcgg cgaaggaaca tccccactcg ctgcatgaca 60

cacggttcga gtaagtgcaa tct 83

<210> 20
<211> 83
<212> DNA
<213> Artificial

<220>
<223> aptamer

<400> 20
gggagatacc agctattcat tccctggcgg cgaaggaact tccccactcg ctgcatgaca 60

cacggttcga gtaagtgcaa tct 83

<210> 21
<211> 81
<212> DNA
<213> Artificial

<220>
<223> aptamer

<400> 21
gggagatacc agctattcat tccctggcgg cgaaggaact tccccactcg ctgcatgaca 60

cggttcgagt aagtgcaatc t 81

<210> 22
<211> 81
<212> DNA
<213> Artificial

<220>
<223> aptamer

<400> 22
gggagatacc agctattcat tccctggcgg cgaaggaact tccccactcg ctgcgtgaca 60

cggttcgagt aagtgcaatc t 81

<210> 23
<211> 81
<212> DNA
<213> Artificial

<220>
<223> aptamer

<400> 23
gggagatacc agctattcat tcccgcgtat aaaggcccaa cactgtggtg ttctgccgcg 60

ccgttcgagt aagtgcaatc t 81

<210> 24
<211> 80
<212> DNA
<213> Artificial

<220>
<223> aptamer

<400>  24
gggagatacc agctattcat tccataggac gagcgggtcc ggtcctgatg gtatccttac      60

tgttcgagta agtgcaatct                                                  80


<210>  25
<211>  80
<212>  DNA
<213>  Artificial

<220>
<223>  aptamer

<400>  25
gggagatacc agctattcat tcagtgcact gatggcccct ggaacccaca cctgggagtt      60

ggttcgagta agtgcaatct                                                  80


<210>  26
<211>  80
<212>  DNA
<213>  Artificial

<220>
<223>  aptamer

<400>  26
gggagatacc agctattcat tccgtgcgac gcaaaaattt gcccacgacc acagtgtccg      60

ggttcgagta agtgcaatct                                                  80


<210>  27
<211>  80
<212>  DNA
<213>  Artificial

<220>
<223>  aptamer

<400>  27
gggagatacc agctattcat tcagtgcact gattgcccct ggaacccaca cctgggagct      60

ggttcgagta agtgcaatct                                                  80


<210>  28
<211>  80
<212>  DNA
<213>  Artificial

<220>
<223>  aptamer

<400>  28
gggagatacc agctattcat tcagtgcact gatagcccct ggaacccaca cctgggagtt      60

ggttcgagta agtgcaatct                                                  80


<210>  29
<211>  80
<212>  DNA
<213>  Artificial

<220>

<223>  aptamer

<400>  29
gggagatacc agctattcat tcagtgcact gatggcccct ggaacccata cctgggagtt      60

ggttcgagta agtgcaatct                                                  80


<210>  30
<211>  80
<212>  DNA
<213>  Artificial

<220>
<223>  aptamer

<400>  30
gggagatacc agctattcat tccaactccc cccaatggta tccttacacg accgcattcg      60

ccttcgagta agtgcaatct                                                  80


<210>  31
<211>  80
<212>  DNA
<213>  Artificial

<220>
<223>  aptamer

<400>  31
gggagatacc agctattcat tcccggcaca ctggtatcct tacacgtccc cattctccct      60

ggttcgagta agtgcaatct                                                  80


<210>  32
<211>  81
<212>  DNA
<213>  Artificial

<220>
<223>  aptamer

<400>  32
gggagatacc agctattcat tcccaaagcg cgccggtatc cttactcgct gtcttcccaa      60

ccgttcgagt aagtgcaatc t                                                81


<210>  33
<211>  80
<212>  DNA
<213>  Artificial

<220>
<223>  aptamer

<400>  33
gggagatacc agctattcat tctagtgccc catggccagc ccccaagcca tgcgtcctgt      60

gcttcgagta agtgcaatct                                                  80


<210>  34
<211>  80
<212>  DNA
<213>  Artificial

<220>
<223>  aptamer

<400>  34
gggagatacc agctattcat tcagcctcgc cgctacgcat tcccaagctg ccgcgcctct      60

ggttcgagta agtgcaatct                                                  80


<210>  35
<211>  80
<212>  DNA
<213>  Artificial

<220>
<223>  aptamer

<400>  35
gggagatacc agctattcat tcccgaaggc cggagtgggc aaaaaacatc ccgctcgctt      60

cattcgagta agtgcaatct                                                  80


<210>  36
<211>  80
<212>  DNA
<213>  Artificial

<220>
<223>  aptamer

<400>  36
gggagatacc agctattcat tccccagttg ggcggaaatc cctaacccaa ctacccaggc      60

ggttcgagta agtgcaatct                                                  80


<210>  37
<211>  80
<212>  DNA
<213>  Artificial

<220>
<223>  aptamer

<400>  37
gggagatacc agctattcat tccgtgcgac gcaaaaattt gcccacgacc acagtgtccg      60

ggttcgagta agtgcaatct                                                  80


<210>  38
<211>  82
<212>  DNA
<213>  Artificial

<220>
<223>  starting aptamer oligo


<220>
<221>  misc_feature
<222>  (19)..(58)
<223>  n is a, c, g, or t

<400>  38
agattgcact tactcgaann nnnnnnnnnn nnnnnnnnnn nnnnnnnnnn nnnnnnnngg      60

aatgaataag ctggtatctc cc 82

## Description

The invention relates to aptamers which suppress metastatic cancer cell migration and invasion, as well as methods of creating and improving such aptamers and the use of such aptamers in research, diagnostic and clinical applications.

Metastasis is the main factor in cancer mortality. Metastatic tumour cells are able to spread throughout the body and grow at distant sites increasing morbidity. Despite progress in oncology, there is still a lack of therapies that can efficiently block metastasis. If it was possible to prevent cancer cells from spreading, primary tumours would remain confined and could be more easily cured by surgical removal or other therapies.

Cell migration plays a crucial role in metastatic spread. The targeting of cell motility and invasiveness is considered as a promising alternative to more traditional methods of therapeutically treating tumours. Tumour cell migration and invasion within a three-dimensional tissue matrix are complex processes that include mobility of individual cells as well as collective cell movements and comprise diverse cellular and molecular mechanisms. It has turned out to be difficult to block cell migration effectively with pharmacological compounds. Current strategies aim at interrupting the cell-extracellular matrix contacts (Sawyer, 2004; Tucker, 2003) or reducing protease activity of tumour cells (Zucker et al., 2000; Overall and Lopez-Otin, 2002; Coussens et al., 2002). Unfortunately, none of these compounds has reached the market for various reasons such as poor *in vivo* anti-tumour activity, an unsuitable therapeutic index or the fact that tumour cells develop mechanisms to overcome the activity of these drugs (Zucker et al., 2000; Friedl and Wolf, 2003).

The majority of current anticancer treatments target tumour cells' ability to proliferate. However, this type of therapy has a limited success rate. Some cells can stay dormant for a long period before generating new tumours (Naumov et al, 2002, Entschladen, 2004). Moreover, migrating cells are able to decrease their proliferation rate, becoming less sensitive to traditional chemotherapy, the most common form of antiproliferative treatment (Douma et al, 2004; Giese et al, 2003; Haga et al, 2003). A major aim of modem anticancer strategies is to inhibit or at least to reduce the dissemination of tumour cells by targeting factors or markers regulating their migratory activity.

In modem drug development, the identification of medicaments which specifically inhibit protein function, whilst not affecting more general cellular or biological progresses is recognized as being an important feature. Targeting of cancer-specific macromolecules within tumour tissues should be more beneficial as opposed to traditional drugs, which tend to be toxic for both tumour and normal cells. Several classes of inhibitors have been employed, including monoclonal antibodies, peptides and small molecules.

The search for ligands/molecules capable of recognizing and interfering with the function of a molecular determinant or marker associated with a specific type of tumor, or else a given stage in its development, or alternatively signaling the metabolic state of a tumor, is therefore essential for better research, diagnosis and therapy of cancers. Unfortunately, these ligands are generally obtained from targets which have been purified and isolated out of their biological context, and which are therefore different from the targets placed in their natural environment.

Thus, most commonly, the ligands, which are effective *in vitro,* are incapable of interacting with their target:
- because they cannot cross tissue barriers,
- because they are instable in the organism, or responsible for too many adverse interactions with other biomolecules,
- because the natural structure of the target, placed out of its natural cell environment, was not conserved or because certain essential modifications of this structure, such as: (i) post-translational modifications of proteins or (ii) interactions with other proteins, cannot be reproduced *in vitro.*

The latter two limitations are particularly frequent in the case of targets such as transmembrane proteins comprising a lipophilic segment inserted into the lipid cell membrane; this lipophilic segment is not conserved *in vitro*, whereas the membrane insertion of these proteins determines their structure and is essential to their activity.

Furthermore, the problem arises of the specificity with which the available ligands recognize targets identified in tumors. It is therefore important to be able to provide specific ligands for diagnosing and/or treating certain cancers, in particular those related to metastasis.

Molecular medicine therefore needs new molecular recognition probes which are:
- specific,
- adjustable, and
- easy to produce at a reasonable cost.

Pharmacological research has set up novel strategies for discovering novel ligands effective against targets identified in tumors:
- combinatorial libraries of small molecules make it possible to increase the chances of finding a ligand against a specific protein (example: a subclass of combinatorial libraries, false substrates which inhibit enzymes, such as those which inhibit MMPs (matrix metalloproteases)). Their major drawback is that they are sorted *in vitro.* Moreover, their selectivity is not guaranteed, hence the difficulty in obtaining, with these agents, a compound which is sufficiently selective to be effective and free of side effects. Only exceptionally are compounds obtained which are specific for an abnormal protein form, which leads to non-specific binding and adverse effects and results in a poor therapeutic index;
- monoclonal antibodies are excellent agents for specific targeting and have recently been used for therapeutic purposes (example: Herceptin in breast cancer); however, they remain very difficult to use *in vivo* due to their size, idiotypy and inherent immunogenicity. They are also extremely expensive to produce and to optimize. Furthermore, monoclonal antibodies reach their limits when the recognition of a point mutation affecting a single amino acid on a protein is involved.;
- aptamers, which constitute an alternative means of diagnosis and therapy, and which have a certain number of advantages compared with antibodies, as illustrated in Table I below.

**Table I**

| | **Aptamers** | **Antibodies** |
|---|---|---|
| Size | 9-15 kDa | >150 kDa |
| discrimination* | +++ | +/++ |
| affinity | 5-100 nM | 0.1-100 nM |
| Source | synthetic | Animal/Animal Cell Culture |
| Cost | ↘ | ↗ |

| | | |
|---|---|---|
| *theophylline versus caffeine, for example. | | |

Technological improvements have made the novel class of potential inhibitors aptamers more readily available. Engineered aptamers are structured small oligonucleotides that bind tightly and specifically to a target molecule just like protein antibodies (Jayasena, 1999). In the case of proteins, high affinity oligonucleotide aptamers often inhibit function, presumably by interactions that overlap the binding site of natural ligands. Some properties of aptamers make them very attractive potential therapeutic agents, as compared to antibodies. Firstly they seem to lack immunogenicity in man (Eyetech Study Group, 2002), secondly they can be chemically modified for improved stability in body fluids and thirdly, due to there small size, are able to penetrate tumours more readily.

Aptamers of various types have previously been used for tumour imaging, diagnostic and, to a limited extent, cancer treatment. G-rich aptamers were demonstrated to have an antiproliferative effect *in vitro* and activity against human tumour xenografts *in vivo* (Bates et al, 1999). These G-rich aptamers consisted only of deoxy- guanidine and thymidine and contained runs of at least two guanosine residues. No selection system was used to generate these aptamers or improve there effects. Such G-rich aptamers were found to inherently inhibit tumour cell proliferation by an interaction with a membrane bound G-rich oligonucleotide binding protein, thought to be nucleosin. Such aptamers are not therefore generally useful in the visualisation or treatment of cancer cells as there effects upon these cancer cells was found to be an effect of the polynucleotide structures they formed due to there G-rich nature rather than any engineered affinity for a suitable anticancer marker.

The TT1 aptamer to tumour matrix protein tenascin-C was reported to be taken up by a variety of solid tumours (Hicke et al, 2006). The TT1 aptamer was isolated using SELEX, see below, to tumour cells expressing tenascin-C and purified tenascin-C. The problems inherent in the generation of materials reactive with isolated tumour cell markers as outlined above therefore apply. In addition such an aptamer and methods of generating it are limited to identifying aptamers to those genes which have previously been characterized as being involved in cancer cell development/differentiation such as tenascin-C.

Aptamers can be created by the combinatorial method for aptamer generation called SELEX (Systematic Evolution of Ligands by EXponential enrichment) (Tuerk and Gold, 1990, Ellington and Szostak, 1990).

Briefly, the principle of the SELEX method involves the selection, from a mixture of nucleic acids comprising random sequences, and by successive reiterations of binding, separation and amplification steps, of nucleic acid molecules (aptamers) exhibiting a defined binding affinity and a defined specificity for a given target. Thus, starting from a mixture of random nucleic acids, the SELEX method comprises more specifically the following steps:
- bringing the mixture of nucleic acids into contact with the target element (natural or synthetic polymers: proteins, polysaccharides, glycoproteins, hormones, receptors, antigens, antibodies, cell surfaces; small molecules: medicament, metabolites, cofactors, substrates, transition state analogs; tissues and in particular whole cells; viruses, etc.), under conditions which promote binding,
- separating the unbound sequences,
- dissociating the nucleic acid-target element complexes,
- amplifying the dissociated nucleic acids, so as to obtain a mixture enriched in ligands (aptamers), and
- repeating the binding, separation, dissociation and amplification steps, for the desired number of times.

High-affinity and high specificity aptamers are selected from a vast random sequence oligonucleotide library (normally 10¹⁴ initial molecules). Evolution is directed by the reduction of initial pool complexity during reiterative cycles of physical separation of target-binding sequences from inactive variants, followed by their reamplification. Aptamers can theoretically be developed to any target, from a pure single molecule to complex systems like a whole cell (K.N. Morris, et al 1998, M. Blank, et al, 2001, Daniels, et al, 2003, Cherchia et al, 2005). The binding of an aptamer strongly depends on the conformational state of its target (Tuerk and Gold 1990).

Recently subtractive SELEX has proved to be a useful tool in finding ligands to specific biological markers that distinguish a subtype of cells from cells of homologous origin. This strategy was used for the isolation of aptamers capable of distinguishing differentiated PC 12 cells from normal PC 12 cells (Wang J et al. 2003), and tumour vasculature from those of the normal brain (Blank et al, 2001).

The Inventors have also previously used a modified SELEX method to identify aptamers able to recognise or act as ligands to membrane receptors with tyrosine kinase activity, e.g. Receptor protein-tyrosine Kinases (RTKPs), (Wo05/093097). The Inventors were able to isolate aptamers specific for RTKPs which both bound to these RTKPs and inhibited the activity of the bound RTKP and also aptamers which only bound to the RTKP without affecting there activity. To do this the Inventors introduced specific activating mutations into the coding sequence of the RET (Rearranged During Transfection) receptor gene. The RET oncogene encodes an abnormal form of a receptor-type surface protein of the tyrosine kinase family; this protooncogene is located on chromosome 10q11.2. Mutations in the Ret protooncogene are associated with several diseases, in particular Hirschsprung's disease and multiple endocrine neoplasia type II (or MEN 2), which includes MEN type 2A (MEN 2A), MEN type 2B (MEN 2B) and familial medullary thyroid cancer (or FMTC).

The RET gene/protein in Wo05/093097 had been activated by mutation at a cysteine located in the extracellular domain, preferably at codons 609, 611, 618, 620 or 634. The coding sequence of this mutated gene was then transfected into a mammalian cell line and a modified SELEX method was performed using such transfected cells as the target element. This system also comprised a intermediary step of exposing the nucleic acid molecules to cells transformed with RET modified with an intracellular mutation, so further increasing the specificity of the aptamers to recognize only activated RET which is the oncogenic form of this protein.

Such a system however is limited to identifying aptamers to those genes which have previously been characterized, as in the case of the proto-onco gene RET, as being involved in cancer cell development/differentiation and also requires the cloning, suitable alteration of such a gene coding sequence and its transfection into cells so as to create the required cancer phenotype as a target element, against which aptamers can be generated.

Given the useful properties known for aptamers the Inventors have continued to pursue further means of using these molecules in the characterisation, diagnosis and potential treatment of cancer cells which are cancerous due to both known and unknown genetic changes.

In particular the Inventors have now developed a new system to identify aptamers which target metastasis specific markers and in some cases also block the metastatic spreading of these cells making all such aptamers useful as research and diagnostic tools and a subset of these also potentially useful as anti-metastatic cancer agents.

The Inventors have used a new modified SELEX approach to identify aptamers that distinguish high metastatic cells from isogenic non-metastatic cells. They have also shown that some of the aptamers they have generated can block metastatic cell migration and/or cell invasion.

Therefore the present invention relates to a method for identifying aptamers specific for at least one metastatic cancer cell marker, using a mixture of nucleic acids, wherein said method comprises at least the following steps:
(a) bringing a mixture of nucleic acids which form a combinatorial library into contact with cells not expressing said at least one metastatic cancer cell marker (C_{N} Cells), said C_{N} cells being the same cell type as metastatic cells expressing said at least one metastatic cancer cell marker (C_{M} cells);
(b) recovering a first subset S 1 of nucleic acids which do not bind to the C_{N} cells, in step (a);
(c) bringing the first subset S1 into contact with the C_{M} cells;
(d) recovering the nucleic acids which exhibit a high affinity with respect to the cells expressing said at least one metastatic cancer cell marker expressed by said C_{M} cells, after dissociation of the cell-nucleic acid complexes;
(e) amplifying said nucleic acids with high affinity for the cells expressing said at least one metastatic cancer cell marker, so as to obtain a mixture of nucleic acids, enriched in nucleic acids having a high affinity for said C_{M} cells, and
(f) identifying aptamers specific for the cells expressing said at least one metastatic cancer cell marker, from the mixture obtained in (e).

Such a method is particularly advantageous as unlike prior art methods the current method allows the generation of the aptamers against specific metastatic cancer cell markers without the need to choose beforehand therapeutically meaningful target molecules. The use of the modified SELEX method developed by the Inventors can generate high affinity aptamers for multiple targets within a complex system such as a metastatic cancer cell in which numerous different corrupted cellular mechanisms all contribute to the disease state. In particular the use of this modified SELEX method has been shown to generate functional aptamers to molecules participating in the achievement of migratory phenotype associated with metastatic cancer cells and responsible for the more deleterious aspects of such cancer cells when these occur in a patient.

In anti-cancer drug discovery, selection of aptamers by intact cancerous cells has some indisputable advantages. Particularly it does not require a full understanding of the complex mechanisms of the cellular features targeted. Thus, there is no necessity to decide which molecular target will have therapeutic importance at the outset of the study.

This method is not target-based, that is individual identified targets are not the basis for the selection process nor is it necessary to select these targets prior to the beginning of the selection process. Selection is instead made against a complex phenotype comprising and resulting from a multitude of interacting genes and gene products. This is an important difference as there has been a perceived failure in the field of target-based drug discovery approaches in the post-genomic era which has lead to a renewed interest in and renaissance of techniques which allow the screening of collections of compounds against complex phenotypes with the objective of eliciting a specific desirable effect.

The invention relies upon the comparison of two or more isogenic cell lines with different metastatic potential.

Therefore starting with two or more isogenic cancer cell lines with different metastatic potential, this system allows the selection of aptamers with anti-metastatic activity and/or more generally the selection of aptamers which specifically recognise metastatic cells.

In particular the method is characterized in that steps (a) to (f) are repeated using the mixtures enriched in aptamers from the preceding cycle, until at least one aptamer is obtained, the affinity of which, defined by its dissociation constant (Kd), can be measured and is suitable for pharmaceutical use.

In particular the method is characterized in that steps (a) to (f) are repeated several times.

Preferably steps (a) to (e) are repeated between at least 10 times and 15 times.

In particular the method is characterized in that the starting nucleic acid combinatorial library contains at least 10⁹ nucleic acids, preferably between 10¹² and 10¹⁶ nucleic acids, and advantageously consists of nucleic acids comprising random sequences flanked, respectively at their 5' and 3' ends, by fixed sequences for PCR amplification, preferably the sequences SEQ ID NO:1 and SEQ ID NO:2 or a fragment of at least 8 nucleotides of these sequences.

In particular the method is characterized in that said random sequences each contain between 10 and 1000 nucleotides, preferably 40 nucleotides, and comprise deoxyribonucleic acid, ribonucleic acid or modified nucleic acids.

In particular the method is characterized in that the identification of the aptamers specific for the C_{M} cells according to step (f) comprises an evaluation of the biological activity of said aptamers on said C_{M} cells.

In particular the method is characterized in that said biological activities which are evaluated is selected from the following:
(a) inhibition or activation of horizontal cell migration,
(b) inhibition or activation of vertical cell migration,
(c) inhibition or activation of cell invasion.

Parallel screening of the aptamers isolated with affinity for the metastatic cell line, for a particular effect of interest such as reducing cell migration in a suitable *in vivo* or *in vitro* assay allows the identification of aptamers which are both specific to the metastatic cell line and also have the desired biological effect.

The present invention also relates to an aptamer, characterized in that it is specific for cells expressing at least one metastatic cancer cell marker, and can be identified by means of the method for identifying aptamers as described in the current application.

Several categories of potential targets or markers include:
- Molecules with an established role in metastasis, such as Growth Factors and Receptors: CSF1 (csf-1), CSF1R (c-fms/MC-SF-R), FGF1 (a-FGF), FGF2 (b-FGF2), HGF (Scatter factor), IGF2, NGFB, PDGFA, TGFA (TGF-a), TGFB1 (TGF-b 1), VEGF, VEGFC; Cell-Cell and Cell-Matrix Interaction Molecules: CAV1 (caveolin-1), CDH1 (cadherin-1/ E-cadherin), COL4A2 (collagen a₂(IV)), ICAM5 (telencephalin), ITGA2, ITGA3, ITGA5, ITGA6, ITGB1, ITGB3, LAMB1 (laminin b1), LAMC1 (laminin b 2), MICA (MUC-18), MUC1, NCAM1, PECAM1, VTN (vitronectin); Metastasis-Associated Proteases: Matrix Metalloproteinases: MMP1, MMP2, MMP3, MMP7, MMP8, MMP9, MMP10, MMP11, MMP13, MMP14, MMP15, MMP16; Others: CASP8, CASP9, CST3 (cystatin C), CTSB (cathepsin B), CTSD (cathepsin D), CTSL (cathepsin L), ELA2 (elastase), HPSE (heparanase), MGEA5 (meningioma hyaluronidase 5), PLAU (uPA), TMPRSS4; Protease Inhibitors: SERPINB2 (PAI-2), SERPINB5 (maspin), SERPINE1 (PAI-1), THBS1, THBS2, TIMP1, TIMP2, TIMP3; Signal Transduction Molecules: LIMK1 (LIM kinase), PLAUR (uPAR), PIK3C2B, RAC1; Oncogenes: ERBB2 (c-erb-2/neu), ETS1 (c-ets-1), ETS2 (c-ets-2), ETV4 (PEA3), FES, FOS (c-fos), HRAS (c-hRas), MDM2, MYC (c-myc), RAF1, SRC (c-src); Metastasis Suppressors: BRMS1 (BrMS1), CD44, DCC, KAI1, KISS1 (KiSS-1), MAP2K4 (mkk4 (JNKK1), MTA1, NM23A (NM23), NME4, PTEN; Other Related Genes: API5 (apoptosis inhibitor 5), ARHC (Rho C), EHM2, ENPP2 (autotaxin/ ATX), MGAT3 (acetylglucosaminyltransferase III), MGAT5 (acetylglucosaminyltransferase V), ODC1, PTGS2 (cox-2), S100A4 (mts-1), SNCG (BCSG1), SPP1 (osteopontin).
- Or genes with no established role in metastasis, such as genes with unknown or putative functions, but against which the current method has been able to raise specific aptamers implicating these genes in this disease phenotype.

The above list of potential target for the aptamers of the current invention is intended to be non-limiting.

In particular the aptamer is characterized in that it recognises at least one metastatic cancer cell marker and in particular at least one metastatic cancer cell marker induced by infection of said cell by an oncovirus.

In particular the aptamer is characterized in that said oncogenic virus is Rous sarcoma virus.

In one example of the present invention cells are infected with Rous sarcoma virus with the oncoviral gene v-src (viral-src) transforming the cells into a metastatic state. In general the transforming gene v-src of Rous sarcoma virus, which has a tyrosine specific protein kinase activity, is sufficient to transform cells into a metastatic phenotype. The exact mechanism of such transformation is still unclear; however it appears to play an important role in aspects of tumour progression, including proliferation, disruption of cell/cell contacts, migration, invasiveness, resistance to apoptosis and angiogenesis. The cellular homolog of v-src, c-src is expressed and activated in a large number of human malignancies and has been linked to the development of cancer and progression to metastases.

Wild type Rous sarcoma virus has been shown to be highly metastatic and in Tatosyan et al (21), a number of naturally occurring variants of the v-src gene were characterized which induce differing levels of metastatic activity in transformed cells. In the present application the cell line HETSR was created by transformation of a starting cell line with a low metastatic v-src carrying variant of Rous sarcoma virus; whereas the cell line HETSR1 was created by transformation of an identical cell line with a high metastatic v-src carrying variant of Rous sarcoma virus. These low and high metastatic v-src genes differ from each other in a number of mutations.

Many other types of viruses are known to be involved in oncogenesis and have been characterized much as Rous sarcoma virus. Each of these and engineered variants thereof is also considered to be suitable to put the present invention into practice.

In particular therefore the aptamer is characterized in that it can be identified by using a first cell line transformed with a v-src gene with a low metastatic potential and a second cell line transformed with a v-src gene with a high metastatic potential.

Alternatively two or more isogenic cell lines could be produced by any suitable method including random mutagenesis by a radiation source or a chemical mutagen. Or specific modification by gene knock out, gene knock in or site directed mutagenesis.

In particular the aptamer is characterized in that it can be identified by means of the method comprising:
(a) bringing a mixture of nucleic acids into contact with C_{N} cells infected with an oncovirus and not expressing at least one metastatic cancer cell marker form,
(b) recovering a first subset S 1 of nucleic acids which do not bind to said C_{N} cells, in step (a),
(c) bringing said first subset S1 into contact with C_{M} cells infected with an oncovirus and expressing said at least one metastatic cancer cell marker,
(d) recovering the nucleic acids which exhibit a high affinity with respect to the cells expressing said at least one metastatic cancer cell marker expressed by said C_{M} cells, after dissociation of the cell-nucleic acid complexes;
(e) amplifying said nucleic acids with high affinity for the cells expressing said at least one metastatic cancer cell marker, so as to obtain a mixture of nucleic acids, enriched in nucleic acids having a high affinity for said C_{M} cells, and
(f) repeating steps (a)-(e), until at least one aptamer is obtained, the affinity of which for the C_{M} cells, defined by its dissociation constant (Kd), is measurable and suitable for a pharmacological activity, and
(g) identifying the aptamers specific for the cells expressing at least one metastatic cancer cell marker, selected from the mixture obtained in (f).

The present invention also relates to an aptamer, characterized in that it can be obtained by means of a method of identification as defined in the current application and in that it is selected from aptamers of formula (I):

R₁-R-R₂ (I),

in which:
R₁ represents any nucleotide sequence which is able to be used to amplify the aptamer.

In particular R₁ is 5' AGATTGCACTTACTCGAA 3' (SEQ ID NO:1) or a fragment of 1 to 18 nucleotides of said SEQ ID NO:1;

R₂ represents any nucleotide sequence which is able to be used to amplify the aptamer.

In particular R₂ is 5' GGAATGAATAAGCTGGTATCTCCC 3' (SEQ ID NO:2) or a fragment of 1 to 24 nucleotides of said SEQ ID NO:2, and

R represents a random sequence of 10 to 1000 nucleotides, preferably 40 nucleotides.

SEQ ID NO: 1 and SEQ ID NO: 2 represent examples of sequences for R₁ and/or R₂, which allow PCR amplification of the aptamer. Other sequences which allow the amplification of the aptamer are also encompassed within the present invention.

In particular, the aptamer is **characterized in that** the ribose of each purine have a hydroxyl group or fluorine atom on the carbon in the 2'-position, while the ribose of each pyrimidine have a fluorine atom on the carbon in the 2'-position.

In accordance with the invention, aptamers may be modified such that the riboses of the purines have, as is the case in natural RNA, a hydroxyl (OH) function on the carbon in the 2'-position, while the riboses of the pyrimidines have a fluorine atom on the carbon in the 2'-position. This modification of the 2'-position is known to confer on the nucleic acids a greater resistance with respect to nucleases.

In particular the aptamer is characterized in that it is selected from the following sequences listed in Table II below:

**Table II**

| | Aptamers obtained after 10 rounds of subtractive SELEX upon HETSR1 |
|---|---|
| E37 | GGGAGATACCAGCTATTCATTCCAGTGACGACGTCGCCCGGTTGTCTAGCCCGCGATTCACGGTTCGAGTAAGTGCAATCT (SEQ ID NO:3) |
| E10 | GGGAGATACCAGCTATTCATTCCGCAACCAAGTTTACGGAAATACCGGCTATACGCCCGACGGTTCGAGTAAGTGCAATCT (SEQ ID NO:4) |
| A7 | GGGAGATACCAGCTATTCATTCCCGCGTGTCAAACGCCGGGTACCTCCCTTTCTGCGTTGCGGGTTCGAGTAAGTGCAATCT (SEQ ID NO:5) |
| B38 | GGGAGATACCAGCTATTCATTCCTACGCCATAGGACGAGGAATAGGTCCTGAAATCTTGGGCCFTCGAGTAAGTGCAATCT (SEQ ID NO:6) |
| D3 | GGGAGATACCAGCTATTCATTCCCTGGCGGCGAAGGAACATCCCCACTCGCTGCATGACACGGTTCGAGTAAGTGCAATCT (SEQ ID NO:7) |
| D12 | GGGAGATACCAGCTATTCATTCCCTGGCGGCGAAGGAACATCCCCACTCGCTGCATGACACACGGITCGAGTAAGTGCAATCT (SEQ ID NO:8) |
| D42 | GGGAGATACCAGCTATTCATTCCCTGGCAGCGAAGGAACATCCCCACTCGCTGCATGACACGGTTCGAGTAAGTGCAATCT (SEQ ID NO:9) |
| G11 | GGGAGATACCAGCTATTCATTCCGGTACCAAGGCTACCTGAATACCCGACGGATTTTCCGGGTTCGAGTAAGTGCAATCT (SEQ ID NO:10) |
| 6/10 | GGGAGATACCAGCTATTCATTCCAGCCGCTGTAATGCGTTCATCCCATTGCCCCGCGCTGGTTCGAGTAAGTGCAATCT (SEQ ID NO:11) |
| **8**/10 | GGGAGATACCAGCTATTCATTCCAAGCACATTCGCTTCGGAACTTCAGCCTGACTCTTGGCTGTTCGAGTAAGTGCAATCT (SEQ ID NO:12) |
| 12/10 | GGGAGATACCAGCTATTCATTCCCCGGCACACTGGTATCCTTACACGTCCCCATTCCCCCTGGTTCGAGTAAGTGCAATCT (SEQ ID NO:13) |
| 15/10 | GGGAGATACCAGCTATTCATTCCTAGAGCCCTGCACTAGTGCACGTCCAGCTGTAGCAGCGGGTTCGAGTAAGTGCAATCT (SEQ ID NO:14) |
| 18/10 | GGGAGATACCAGCTATTCATTCCTGATGTGCAGACGCTCGTGGCTTAGGCCATCACATCCGGGTTCGAGTAAGTGCAATCT (SEQ ID NO:15) |
| 33/10 | GGGAGATACCAGCTATTCATTCCCACCTTCCTGGTATCCTTACTCGTAACAGCCCGTCTCCCGTTCGAGTAAGTGCAATCT (SEQ ID NO:16) |
| 34/10 | GGCAGATACCAGCTATTCATTCCGGCGACACCTGCCAGCTCCCGACATCGTGTGAAAACTGCGCTTCGAGTAAGTGCAATCT(SEQ ID NO:17) |
| 35/10 | GGGAGATACCAGCTATTCATTCCTAGTGCCCCATGGCCAGCCCCCGAGCCATGCGTCCTGTGCTTCGAGTAAGTGCAATCT (SEQ ID NO:18) |

| | Aptamers obtained after 5 further rounds of subtractive SELEX upon HETSR1 |
|---|---|
| D11 | GGGAGATACCAGCTATTCATTCCCTGGCGGCGAAGGAACATCCCCACTCGCTGCATGACACACGGTTCGAGTAAGTGCAATCT (SEQ ID NO:19) |
| D35 | GGGAGATACCAGCTATTCATTCCCTGGCGGCGAAGGAACTTCCCCACTCGCTGCATGACACACGGTTCGAGTAAGTGCAATCT (SEQ ID NO:20) |
| D38 | GGGAGATACCAGCTATTCATTCCCTGGCGGCGAAGGAACTTCCCCACTCGCTGCATGACACGGGTTCGAGTAAGTGCAATCT (SEQ ID NO:21) |
| D40 | GGGAGATACCAGCTATTCATTCCCTGGCGGCGAAGGAACTTCCCCACTCGCTGCGTGACACGTTCGAGTAAGTGCAATCT (SEQ ID NO:22) |
| E1/5 | GGGAGATACCAGCTATTCATTCCCGCGTATAAAGGCCCAACACTGTCGTGTTCTGCCGCGCCGTTCGAGTAAGTGCAATCT (SEQ ID NO:23) |
| F18 | GGGAGATACCAGCTATTCATTCCATAGGACGAGCGGGTCCGGTCCTGATGGTATCCTTACTGTTCGAGTAAGTGCAATCT (SEQ ID NO:24) |
| L9 | GGGAGATACCAGCTATTCATTCAGTGCACTGATGGCCCCTGGAACCCACACCTGGGAGTTGGTTCGAGTAAGTGCAATCT (SEQ ID NO:25) |
| 3/15 | GGGAGATACCAGCTATTCATTCCGTGCGACGCAAAAATTTGCCCACGACCACAGTGTCCGGGTTCGAGTAAGTGCAATCT (SEQ ID NO:26) |
| L6 | GGGAGATACCAGCTATTCATTCAGTGCACTGATTGCCCCTGCAACCCACACCTGGCAGCTGCTTCGAGTAAGTGCAATCT (SEQ ID NO:27) |
| L16 | GGGAGATACCAGCTATTCATTCAGTGCACTGATAGCCCCTGGAACCCACACCTGGGAGTTGGTTCGAGTAAGTGCAATCT (SEQ ID NO:28) |
| L31 | GGGAGATACCAGCTATTCATTCAGTGCACTGATGGCCCCTGGAACCCATACCTGGGAGTTGGTTCGAGTAAGTGCAATCT (SEQ ID NO:29) |
| K29 | GGGAGATACCAGCTATTCATTCCAACTCCCCCCAATGGTATCCTTACACGACCGCATTCGCCTTCGAGTAAGTGCAATCT (SEQ ID NO:30) |
| 21/15 | GGGAGATACCAGCTATTCATTCCCGGCACACTGGTATCCTTACACGTCCCCATTCTCCCTGGTTCGAGTAAGTGCAATCT (SEQ ID NO:31) |
| 33/15 | GGGAGATACCAGCTATTCATTCCCAAAGCGCGCCGGTATCCTTACTCGCTGTCTTCCCAACCGTTCGAGTAAGTGCAATCT (SEQ ID NO:32) |
| 32/15 | GGGAGATACCAGCTATTCATTCTAGTGCCCCATGGCCAGCCCCCAAGCCATGCGTCCTGTGCTTCGAGTAAGTGCAATCT (SEQ ID NO:33) |
| 34/15 | GGGAGATACCAGCTATTCATTCAGCCTCGCCGCTACGCATTCCCAAGCTGCCGCGCCTCTGGTTCGAGTAAGTGCAATCT (SEQ ID NO:34) |
| 37/15 | GGGAGATACCAGCTATTCATTCCCGAAGGCCGGAGTGGGCAAAAAACATCCCGCTCGCTTCATTCGAGTAAGTGCAATCT (SEQ ID NO:35) |
| 45/15 | GGGAGATACCAGCTATTCATTCCCCAGTTGGGCGGAAATCCCTAACCCAACTACCCAGGCGGTTCGAGTAAGTGCAATCT (SEQ ID NO:36) |

The invention also relates to a reagent for diagnosing a tumor, characterized in that it consists of an aptamer as described in the current application and in particular one of the aptamers described in Table II above.

The invention also relates to a medicament, characterized in that it comprises an aptamer selected from the group: SEQ ID NO: 3, SEQ ID NO: 4, which has the ability to inhibit metastatic cell migration.

The invention also relates to a pharmaceutical composition, characterized in that it comprises:
an aptamer selected from the group: SEQ ID NO: 3, SEQ ID NO: 4, which has the ability to inhibit metastatic cell migration, and
at least one pharmaceutically acceptable vehicle.

The invention also relates to the use of an aptamer as described in the current application, for screening products which interact with said at least one metastatic cancer cell marker and which affect the migration or invasiveness of a cell expressing said at least one metastatic cancer cell marker.

The invention also relates to a method for screening substances which interact with at least one metastatic cancer cell marker or targets forming a complex with said at least one metastatic cancer cell marker, wherein said method is characterized in that it comprises:
bringing cells expressing said at least one metastatic cancer cell marker into contact with the substance to be tested,
adding, under suitable conditions, an aptamer as defined hereabove, before, at the same time as or after the substance to be tested,
evaluating the competitive binding between said aptamer and said substance to be tested.

In particular the method is characterized in that, after identification of said interacting substance which binds competitively with the aptamer to the cells expressing said at least one metastatic cancer cell marker, the effect of said substance on the biological activity of said cells can be evaluated in order to find substances which inhibit or activate said biological activities of the cells exhibiting said at least one metastatic cancer cell marker.

The invention also relates to a method for identifying at least one molecular target of an aptamer according to any one of the above aptamer types, comprising at least the steps of:
(a) bringing at least one of said aptamer into contact with cells expressing at least one metastatic cancer cell marker expressed by C_{M} cells as defined hereabove;
(b) recovering said at least one aptamer whilst still in complex with said at least one molecular target of said C_{M} cells; and
(c) identifying said at least one molecular target.

The invention also relates to a method for identifying at least one molecular target of an aptamer according to any one of the above aptamer types, comprising at least the steps of:
(a) bringing at least one of said aptamer into contact with cells expressing at least one metastatic cancer cell marker expressed by C_{M} cells as defined hereabove;
(b) recovering the total mRNA content of said cells;
(c) comparing by microarray analysis the mRNA sample of step (b) to that of a control mRNA sample; and
(d) identifying said at least one molecular target.

The invention also relates to a method for identifying at least one molecular target of an aptamer according to any one of the above aptamer types, comprising at least the steps of:
(a) bringing at least one of said aptamer into contact with cells expressing at least one metastatic cancer cell marker expressed by C_{M} cells as defined hereabove;
(b) recovering the total protein content of said cells;
(c) comparing by microarray analysis the protein sample of step (b) to that of a control protein sample; and
(d) identifying said at least one molecular target.
   For a better understanding of the invention and to show how the same may be carried into effect, there will now be shown by way of example only, specific embodiments, methods and processes according to the present invention with reference to the accompanying drawings in which:
   **Figure 1****.** shows the folding of aptamer E10 (SEQ ID NO: 4) (A) and E37 (SEQ ID NO: 3) (B)
   **Figure 2****.** shows the effect of the initial pool (**A**), aptamer E10 (SEQ ID NO: 4) **(**B) and aptamer E37 (SEQ ID NO: 3) (**C**) on migration of HETSR-1 cells into wound.
   **Figure 3****.** shows the binding curves for aptamers E10 (SEQ ID NO: 4) (**A**) and E37 (SEQ ID NO: 3) (**B**).
   **Figure 4****.** shows the binding of aptamers E10 (SEQ ID NO: 4) and E37 to the high (HETSR-1) and low (HETSR) metastatic cell lines.
   **Figure 5****.** shows the effects of the initial pool (**A**), aptamer E10 (SEQ ID NO: 4) (**B**) and aptamer E37 (SEQ ID NO: 3) (**C**) on the migration of HETSR-1 cells.
   **Figure 6****.** shows the effects of initial pool (**A**) and aptamer E10 (SEQ ID NO: 4) (**B**) on invasion of HETSR-1 cells. Quantification of cell invasion shown in (**C**).
   **Figure 7****.** shows volumetric images of living HETSR-1 cells growing in 3D-aggregates, taken after incubation with fluorescent aptamer E10 (SEQ ID NO: 4) (**A**), scramble 10 (**B**), aptamer 37 E10 (SEQ ID NO: 3) (**C**), scramble 37 (**D**).

There will now be described by way of example a specific mode contemplated by the Inventors. In the following description numerous specific details are set forth in order to provide a thorough understanding. It will be apparent however, to one skilled in the art, that the present invention may be practiced without limitation to these specific details. In other instances, well known methods and structures have not been described so as not to unnecessarily obscure the description.

### EXAMPLE 1: MATERIALS AND METHODS

### 1.1 Cell cultures

HET-SR1 and HETSR - cell lines belonging to the cell model that have been already described (Deichman, G. I. et al 1989, Deichman et al 1992). Briefly, they were derived from Syrian hamster embryo fibroblasts after independent transformation in vitro by Schmidt-Ruppin D strain of Rous sarcoma virus. Both lines are highly tumorigenic in animals, but possess different metastatic activity. As a result, xenografts of HETSR-1 cells produce up to 200 lung metastases, whereas HETSR cells usually do not metastasize. Cells were cultured in RPMI 1640, supplemented with 4mM L-glutamine and 10% fetal bovine serum.

### 1.2 Living cell SELEX

The starting pool was composed of 10¹⁴ of body-labelled 2'F-Py RNAs of sequences AGATTGCACTTACTCGAA(N₄₀)GGAATGAATAAGCTG GTATCTCCC (SEQ ID NO: 38) with a central stretch of 40 randomised nucleotides. 2'F-Py signifies that the ribose of each pyrimidine have a fluorine atom on the carbon in the 2'-position.

The oligonucleotides AGATTGCACTTACTCGAA (SEQ ID NO: 1) and GGAATGAATAAGCTGGTATCTCCC (SEQ ID NO: 2) were included at either end of each oligonucleotide in the starting pool, so as to act as primer recognition sites allowing the amplification of selected oligonucleotides which have shown affinity for the target element.

SELEX was performed as described (Cerchia et al, 2005).

A pool of 2'F-Py RNAs (1-5 nM) was heated at 85°C for 5 min in 3ml of RPMI medium, snapped on ice for 5 min and allowed to warm to 37°C.

Counter-selection was performed by a 15 min incubation of the pool with post-confluent HETSR cells at 37°C. After incubation with HETSR cells, the pool was incubated with HETSR-1 cells, which serve as the target element in the selection process. After 15min of incubation at 37°C in the presence of non-specific competitor (50 mg/ml total yeast RNA), unbound sequences were eliminated by several consecutive washes with 5ml RPMI. The remaining RNAs were recovered by phenol extraction, reverse transcribed via specific primers to SEQ ID NO: 1 and SEQ ID NO: 2 and amplified by PCR. PCR-generated double stranded DNA templates were transcribed.

Transcription was performed in the presence of 1mM 2'F-Py and a mutant form of T7 RNA polymerase (T7Y639F). After subsequent gel-purification, the pool of RNA molecules was subjected to the next round of pre-counter-selection/selection. Selective pressure was progressively augmented by increasing the number and time of washings (from one wash for 5 min in the first round to five washes for 10 min each in the last round) and decreasing the number of targeted cells (from 20⁶ to 10⁶ without changing in cell density). Enrichment was followed by monitoring the appearance of four-base restriction sites in the population, which reveals the emergence of distinct families (Bartel and Szostak 1993). After 10 rounds of selection, the pool was cloned with a TOPO TA cloning kit (Invitrogen, Carlsbad, California, United States) and individual clones were analyzed.

### 1.3 Binding experiments

Experiments of binding of individual aptamers or mixed population to HETSR-1 and HETSR cells were performed in 24-well plates at the same cells density as during selection. 5'-P³² labelled RNAs at various concentrations in 200 µl of RPMI medium were incubated with the cells for 15min at 37°C in the presence of total yeast RNA (50mg/ml) as non-specific competitor. After extensive washings (5x500mkl RPMI), the remaining radioactivity was counted and normalized to the number of cells by measuring the protein content in each well.

For binding curves, values of non-specific binding of the scrambled control sequence were subtracted from the aptamer binding values. Dissociation constant (Kd) was determined by Scatchard analysis according to the equation: (bound aptamer)/(aptamer) = -(1/kd)x(bound aptamer)+((T)tot/Kd where (T)ₜₒₜ represents the total target concentration

### 1.4 Wound healing assay

Cells were cultured to postconfluent multilayer in 24-well plates, serum starved for 24h in the presence of 100nM aptamer or initial pool. Wounds were made by scratching the cell multilayer with a pipette tip, followed by rinsing with PBS in order to remove floating or damaged cells. Migration in wounds was stimulated by addition of 5% fetal bovine serum. Cells were allowed to migrate for 9 hours in the presence of 100nM aptamer or initial pool. Wound healing was monitored by phase contrast microscopy.

### 1.5 Transwell migration assay

Cells were serum starved in the presence of 100nM aptamer or initial pool for 24 hours. Migration assays were performed using Boyden chambers with 8 µM pores (Transwell assay system, Costar, Coming Incorporated, USA). 100 000 cells were seeded into the inner chamber of tissue culture inserts Cells were allowed to migrate for 4 hours in presence of 100nM aptamer or initial pool in serum-free RPMI medium towards the outer chamber containing RPMI supplemented with 10% fetal bovine serum. Non-migrated cells were removed from the membrane top with a cotton swab. Cells migrated to the reverse side of membrane were fixed and stained with 20% methanol/0,1% crystal violet for 30 min and then extensively washed with PBS. The total number of cells of the membrane was counted in sets of duplicate.

### 1.6 In vitro Matrigel invasion assay

Cells were serum starved in the presence of 100nM aptamer or initial pool for 24 hours. Inserts with transwell membranes coated with Matrigel matrix (BD Biosciences, San Jose, CA) were used to measure cell invasion in vitro. Cells were plated at 100 000 per insert (upper well) in RPMI containing 100 nM of aptamer or mixed population. Invasion was carried out 20 hours. Medium was aspirated from the top and bottom wells and non-invading cells were removed from the membrane top with a cotton swab. Cells on the reverse side of membrane were fixed and stained with 20%methanol/0,1% crystal violet for 30 min and then extensively washed with PBS. The total number of cells of the membrane was counted in sets of duplicate membranes

**1.7 Fluorescent imaging of living cells** RNA sequences were labelled with Alexa fluor 488 ULS reagent (Invitrogen, Carlsbad, California, United States). Conjugation of aptamers with dyes followed the manufacturer's protocol. Extinction factor and correction factor (absorbance ratio A₂₆₀/Aₘₐₓ for the free Alexa Fluor 488 dye) indicated by the provider allowed to estimate the concentration of the fragments and the amount of dye per fragment. For imaging, cells were plated on Lab-Tek glass slides with chambers (Nalge Nunc International, USA) and allowed to grow to postconfluent multilayer. After 15 min of incubation of fluorescent aptamers or corresponding scrambled sequence at 100nM in RPMI medium, cell were washed three times with RPMI. Images were acquired with a Leica Inverted DMI6000B microscope with filters for FITC (excitation BP450-490, emission BP500-550).

### EXAMPLE 2: RESULTS

### 2.1 Cell-based SELEX

The cellular model that was used for subtractive SELEX was favourable for the generation of potential antimigratory aptamers. For selection and counter-selection two cell lines were used that were obtained as an independent transformation of Syrian hamster embryo fibroblasts by Rous sarcoma virus (Deichman et al., a- 1989, b- 1992). Both were highly tumorigenic in vivo. However, a few C-terminal mutations in transforming viral oncogene v-src determined the remarkable differences of their spontaneous metastatic activity in xenografted Syrian hamsters (Tatosyan et al, 1996). In vitro migratory capacities of low metastatic cells were inferior to those of high metastatic (Isachenko et al, 2006), but their proliferative rates were comparable. Interestingly, the spectrum of cellular proteins that interact with v-src in the high and low metastatic cell lines do not differ significantly (Mizenina et al., 2001).

High metastatic cell line HETSR-1 served for the aptamer selection and the low metastatic HSR cell line served for counter-selection. The repertoire of the starting pool was composed of 10¹⁴ unique sequences of 82-mer 2'-fluoropyrimidine (2'F-Py) nuclease-resistant RNAs with randomized 40-nucleotide inserts flanked by primer binding sites. Selection was driven towards isolation of aptamers against some unidentified factors present on high metastasic cells as oppose to low metastatic.

In order to mimic the condition of solid tumours, cells were grown to postconfluent multilayers with the appearance of aggregates containing round shape cells attached to each others but not anymore to substrate. Low-metastatic cell line HETSR was used for counter-selection to subtract sequences that would bind equally low metastatic cells. Namely, after incubation with the postconfluent low metastatic cells, the free RNA pool was applied to the postconfluent high metastatic cells. After several washings, bound sequences were recovered, amplified and subjected to the next round of enrichment. In order to accumulate only high-affinity aptamers, selective pressure was augmented steadily by increasing washing times and decreasing target numbers. Progress of the selection was monitored by RFLP (restriction fragment length polymorphism analysis). Distinct cleavage products appeared already at 7-th round, indicating a reduction in the pool complexity (data not shown). After 10-th round of selection, the population demonstrated a 10-fold increase in binding to the targeted HETSR-1 cells in comparison with initial pool.

DNA templates from RNA pool of 10th round were cloned. The Inventors obtained 40 clones in all. All clones were picked and sequenced. Sequence analysis showed that altogether 16 different sequences were isolated (upper portion of Table II above). Based on sequence similarity and energy minimised secondary structure analysis, the prediction of secondary and tertiary structure of the aptamers selected was carried out using the RNAstructure software written by David H. Mathews: http://rna.chem.rochester.edu. The algorithm used by this software is based on the searches described in the publication: D.H. Mathews et al., J. Mol. Biol., 1999, 288, 911-940. The same predictions can be obtained using the mfold algorithm, available on the site of the Michael Zuker laboratory: http://bioinfo.math.rpi.edu/∼zukerm/. The algorithm used by this software is also based on the searches described in the publication D.H. Mathews et al., mentioned above.

A majority of the sequences were grouped into five families. Some sequences were highly abundant in their families. Very few sequences were unique and showed no similarity with any of the family members. Relative binding efficiencies for most abundant members of each family were determined: Individual aptamers showed a 6-15 fold increase in binding efficiencies as compared to the initial pool.

After 10 rounds of selection, five more rounds were performed under an increasing selection pressure in which:
(i) The amount of target was reduced five fold;
(ii) The number of washes after binding was increased stepwise from ten washes in the 10^{th} round to twenty washes in the 15^{th} round.

After the 15^{th} round, the complete aptamer population was cloned and all clones were collected and sequenced. The aptamers selected are shown in the lower half of table II above.

Sequence analysis showed a reduction in the number of sequences, representing the most abundant family at the 10^{th} round. Three other families survived selective pressure better and were enriched in the population. One new group of highly similar and very abundant sequences appeared (30% of mixed population). At the same time, several individual new sequences were found. This suggests that the five additional rounds of selection caused new evolutionary changes in the oligonucleotide population and gave rise to new aptamer types.

Analysis of the binding activity of major representatives of all groups showed their capacity to bind highly metastatic cells 6-10 times more strongly than the initial pool. This suggests that sequences obtained after the 15^{th} round of selection are also metastasis-specific aptamers.

### 2.2 Wound healing assay

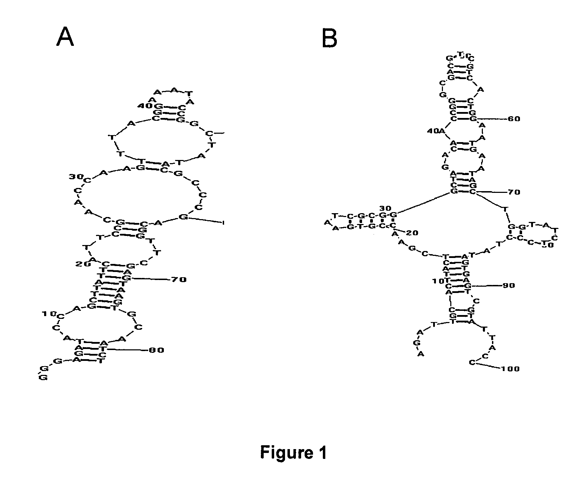

In order to uncover sequences that would modulate migratory properties of HETSR-1 cells, a parallel screen with 17 aptamers was performed, that tested their impact on HETSR-1 cell migration. Wound healing was used as a readout. After wounding a multilayer of postconfluent cells, cell migration and wound healing was monitored at regular intervals in the presence of serum and aptamer. A starvation step was performed in order to slow down proliferation activity, which may contribute to wound closure along with the migration. Initial experiments were performed at 500 nM of each aptamer in triplicate. Only in few cases, we observed an inhibition of cell migration in comparison with the initial pool. Surprisingly, sequences belonging to the largest families and having good binding characteristics did not modulate cell migration. Two aptamers, E10 (SEQ ID NO: 4) and E37 (SEQ ID NO: 3), which were potent inhibitors of cell migration, were taken for further analysis. They did not show similarity neither in sequence, nor in structure (see Fig.1A and B).

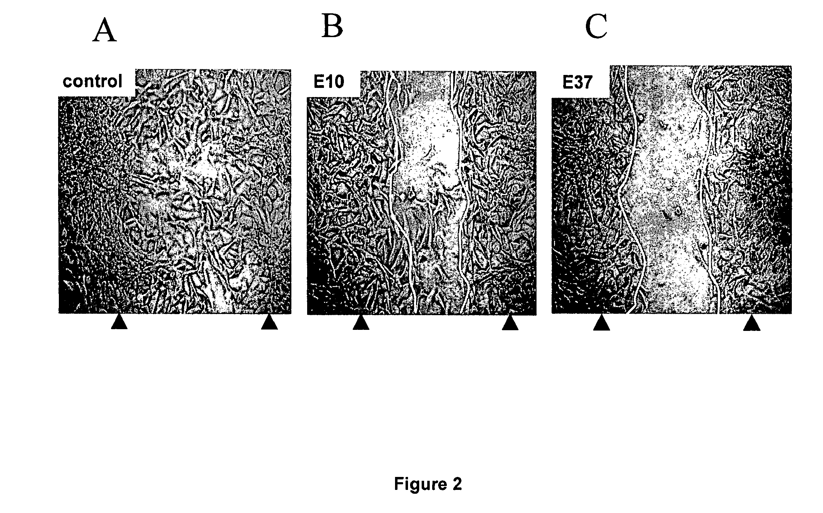

Aptamer E37 (SEQ ID NO: 3) was the most abundant sequence of a small family, whereas E10 was an individual sequence. Fig.2 shows wound closure by cell migration in the case of the initial pool (Fig.2A) and its inhibition by aptamer E10 (SEQ ID NO: 4) (Fig. 2B) and E37 (SEQ ID NO: 3) (Fig. 2C). Wound healing assays were repeated with various concentrations of E10 (SEQ ID NO: 4) and E37 (SEQ ID NO: 3): the effective aptamer concentration could be as low as 50 nM for E10 (SEQ ID NO: 4) and 100nM for E37 (SEQ ID NO: 3). It was necessary to perform serum starvation in the presence of these aptamers, otherwise the observed effect was much weaker. Proliferation tests proved that the inhibition of wound healing by aptamers E10 and E37 concerned cell migration only and was not due to modulation of proliferation rate (data not shown).

In figure 2 arrowheads show the size of initial wound. Before wound healing, cells were incubated with 100nM of corresponding RNA-s overnight in serum-free RPMI medium. Migration was stimulated by addition of 5% of fetal bovine serum and carried out for 9 hours in the presence of aptamer.

### 2.3 Binding affinities of analysed aptamers

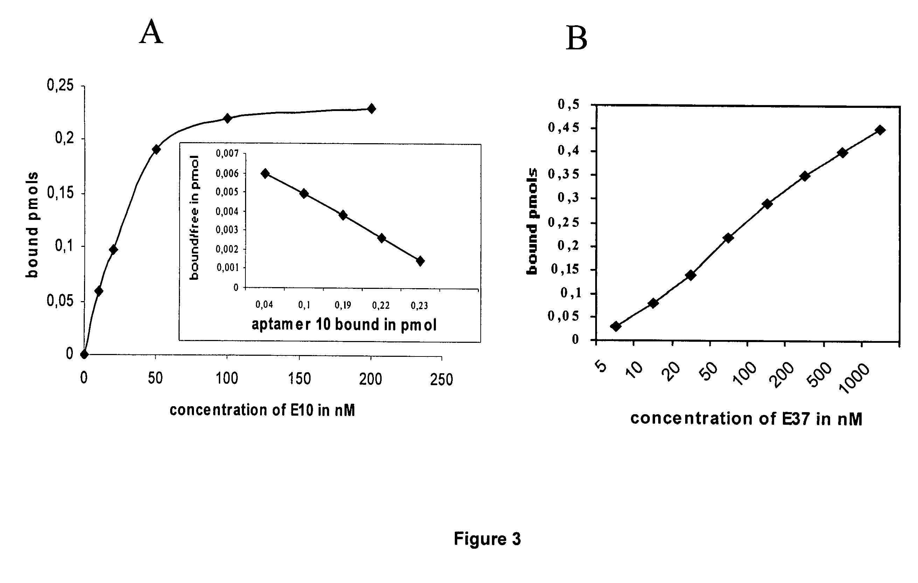

Binding affinities of aptamers E10 (SEQ ID NO: 4) and E37 (SEQ ID NO: 3) were quantified. Different concentrations of radioactively labelled aptamers and corresponding scrambled sequences were incubated with a constant number of cells grown to a post-confluent multilayer. As shown in Fig. 3A, the resulting binding curve of the aptamer E10 (SEQ ID NO: 4) shows saturation at 50 nM, meaning that it has a defined number of binding sites. Its dissociation constant (Kd), calculated by Scatchard analysis was 25 nM, meaning a high affinity interaction. Aptamer E37 (SEQ ID NO: 3) does not saturated target within a 5nM- 1µM concentration range (Fig.3B). However, it binds approximately 10 times stronger than its scrambled sequence (values were subtracted from each data point).

In figure 3 the slope represents -1/Kd. (B) Binding curve, based on different concentrations of E37 (SEQ ID NO: 3). For the binding experiments HETSR1 cells were incubated with increasing concentrations of [³²P]-labelled RNAs at a constant cell density. After five washings, remaining radioactivity was measured. The background binding values for corresponding scrambled sequences were subtracted from each data point.

### 2.4 Binding specificities of analysed aptamers

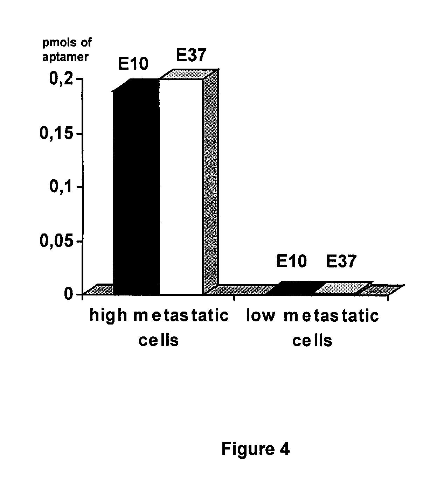

In order to evaluate the capacity of aptamers E10 (SEQ ID NO: 4) and E37 (SEQ ID NO: 3) to distinguish between low and high metastatic cells, binding tests were performed with high metastatic HETSR-1 cells and low metastatic HETSR cells. As shown in Fig.4, both aptamers, E10 (SEQ ID NO: 4) and E37 (SEQ ID NO: 3), bind approximately 10 times stronger to the high metastatic line HETSR-1 than to its low metastatic counterpart. Binding values of both aptamers in the case of low metastatic line HETSR are similar to that of the corresponding scrambled sequences, i.e. at a background level. Thus, both aptamers are highly specific for high metastatic cells HETSR-1 and fail to bind its low metastatic counterpart.

In figure 4 HETSR-1 and HETSR cells were incubated with 50 nM of [³²P]-labelled aptamers. After washing remaining radioactivity was counted and used for the calculation of molar values of aptamers, attached to the cells,

### 2.5 Transwell migration assay

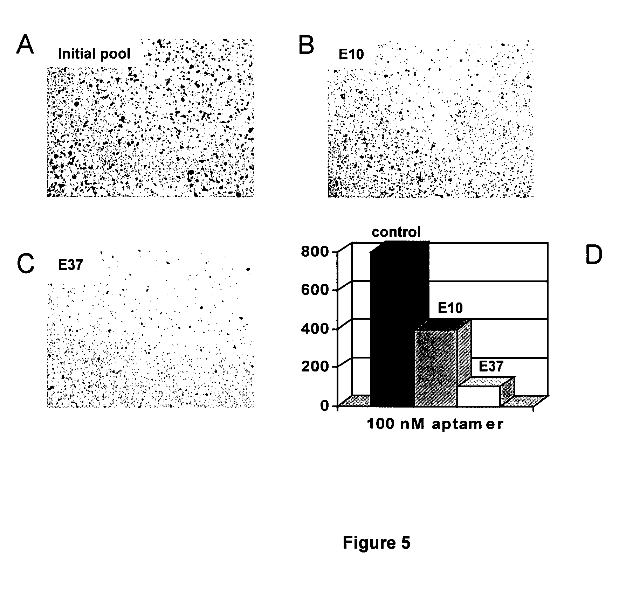

To find out whether E10 (SEQ ID NO: 4) and E37 (SEQ ID NO: 3) can inhibit 3D-dimentional cell migration, we performed a transwell migration assay, which analyses cell plasticity along with cell motility. HETSR-1 cells after overnight starvation in the presence of aptamer or the initial pool were allowed to pass through porous membrane of Boyden chambers. Migrated cells were counted. In agreement with the wound healing experiments, both aptamers suppressed cell migration in this test. In comparison with the initial pool, E37 (SEQ ID NO: 3) caused an 8-fold decrease in the number of migrated cells and E10 a 2-fold decrease (Fig. 5).

In figure 5 quantification of cell migration is shown in (D). Data represents the mean of three independent experiments. Cells were incubated overnight with 100 nM of the mentioned aptamer or of the initial pool in serum-free RPMI. Migration was carried out 4 hours in the presence of 100 nM of the aptamers or of the initial pool. After fixation and staining, all cells having crossed the membrane were counted. P>0,008

### 2.6 Matrigel invasion assay

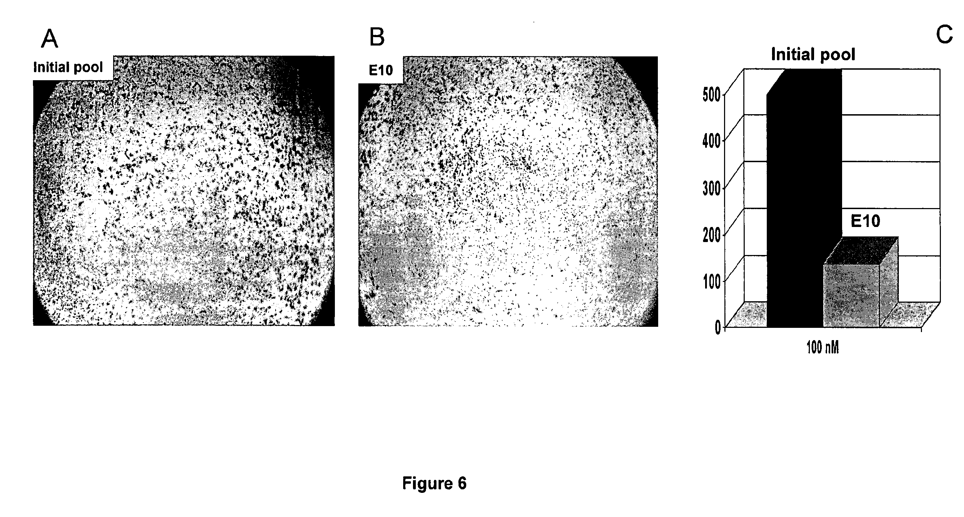

To test whether E10 (SEQ ID NO: 4) and E37 (SEQ ID NO: 3) are able to modulate invasiveness of HETSR-1 cells, we performed a Transwell invasion assay. Here the porous membrane of Boyden chambers are covered with Matrigel matrix, mimicking basal lamina and representing a real barrier for non-invasive cells. Due to the porous membrane of the chambers, the test takes into account cell migration together with cell invasion. HETSR-1 cells were incubated overnight with the chosen aptamers or the initial pool in serum-free medium, then allowed to invade in the presence of aptamers or the initial pool. All cells that degraded the gel matrix and crossed the membrane were counted. Aptamer E10 showed a 4-fold suppression of the invasion of HETSR-1 cells (compare Fig 6A and B) in comparison with the initial pool. Thus, it is more active as anti-invasive aptamer because it decreases cell migration only 2-fold. Interestingly, aptamer E37 (SEQ ID NO: 3), which inhibits migration much stronger than aptamer E10, did not influence cell invasive activity (data not shown).

In figure 6 cells were incubated overnight with 100 nM of aptamers or the initial pool. Invasion was carried out 20 hours in the presence of 100 nM of aptamers or initial pool. After fixation and staining, cells having crossed the membrane were counted. Data represents the mean of three independent experiments. P>0,001

### 2.7 Fluorescent imaging

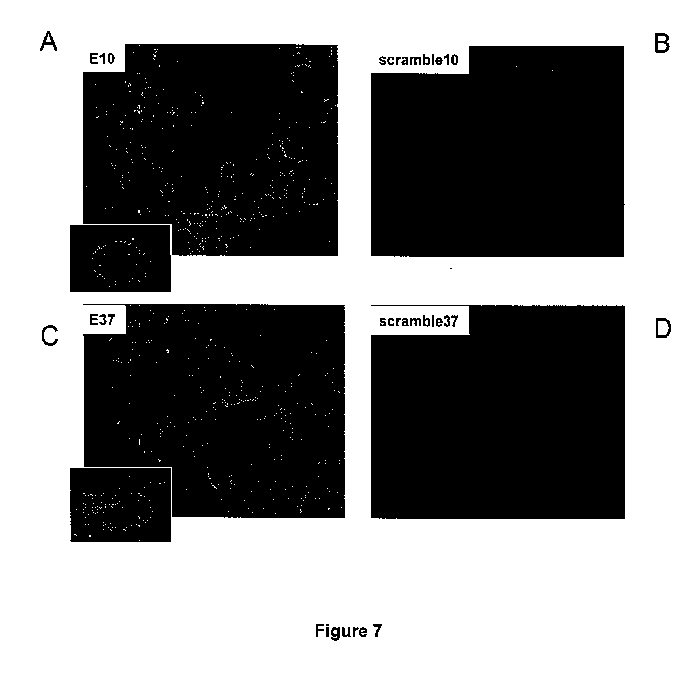

Imaging of living cells incubated with fluorescent aptamers was performed. Time of binding was the same as for the radioactive binding tests and selection experiments. After incubation with fluorescently labelled E10 (SEQ ID NO: 4) or E37 E10 (SEQ ID NO: 3), or corresponding scrambled control sequences, cells were washed from the excess of oligonucleotides, covered slightly with RPMI in order to avoid drying and imaged using a fluorescent microscope. As shown in Fig 6, the binding of both aptamers differs from their scrambled control sequences. The negligible residues of scrambled sequences remaining after washing were uniformly distributed all over cells (Fig. 7C and D). Aptamer E10 was clearly visible and bound to cell surface components, framing round-shape cells. Binding did not show evident preferences for the cell-cell contacts (Fig. 7A). Most surprising was the binding of aptamer E37 (SEQ ID NO: 3): it was visible in the cytoplasm, which means that it penetrated cells, but not into the nucleus. Image of the cell in the insert of Fig 7C shows the fluorescence within the cytoplasm and the dark non-fluorescent nucleus.

In figure 7 RNA-sequences were labelled with Ulysis Alexa Fluor 488 (Invitrogen) and incubated with the cells at 100 nM for 15 min. Images of living cells in RPMI medium were taken after three washes. Each image constructed from 18 stack slices, grouped by Z-projection in software Image J 1.38r (Wayne Rasband National Institutes of Health, USA).

### 2.8 Characterisation of Aptamer Targets

The identification of the molecular targets of the aptamers developed and in particular the targets of aptamers E10 and E37 was then performed. A number of methods are suitable for determining the molecular targets of the aptamers including:
Affinity chromatography;
Transcriptome analysis;
Proteome analysis.

An affinity chromatography based method is a good way of separating the oligonucleotide/protein complexes and so by peptide sequencing the resulting proteins and comparing this to the nucleotide sequence of the oligonucleotide determining the target protein. A potential problem with this approach is that if the target protein is in low abundance it is likely that the small aptamer ligand will be in substantial excess relative to the target and so this could lead to non specific protein binding which in turn could lead to false positives with these non-specifically bound non-target proteins.

Transcriptome or proteome analysis using a micro array system is another approach. The transcriptome is the total mRNA content of a cell and likewise the proteome is the total protein content of a cell. By comparing the levels of transcript or protein in cell populations exposed to the aptamer versus those not exposed it will be possible to identify the transcripts/proteins which are perturbed and hence investigate these further as potential targets of the aptamers.

When the aptamer has been shown to have a specific biological effect such as reducing metastatic cell migration, the differences in transcript/protein levels could prove particularly interesting as at the present time even though metastasis is of great clinical importance the actual mechanisms of metastasis are still not fully understood. Therefore this new technique will also prove to be an important research tool in the fundamental elucidation of complex biological processes such as metastasis which so far have only been partially explained due to their enormously complex and interrelated genetic and biological nature.The design of the subtractive living cell SELEX approach that the Inventors have used is therefore suitable for developing aptamers against factors potentially associated with metastasis and more generally as a means to develop aptamers which are specific to metastasis cells.

The Inventors have shown that the *in vivo* high metastatic cell line of v-src-transformed fibroblasts HETSR-1 can be used as target element in the modified SELEX method. Using a low-metastatic cell line HETSR, derived from the same origin, but transformed with mutated low-metastatic v-src variant, was used for counterselection, the Inventors have shown that specific aptamers were generated from the vast library of random nuclease-resistant RNA-sequences present in the original pool.

As with other SELEX methods the complexity of the pool decreases stepwise by subtracting sequences, able to bind low metastatic cell line and by eliminating non-binders sequences by multiple washes. The use of a low metastatic cell line for counter selection step made this modified system more able to increase the portion of relevant sequences because it leads to elimination of both species and tumour-specific sequences, which are not of interest.

To mimic natural conditions as far as possible, SELEX was carried out on 3-D cultures. In various cell culture applications, it has been shown that the growth and function of cells as multi-cellular 3-D structures is significantly different to their growth as conventional 2-D monolayer cultures (Schmeichel and Bissell, 2003, Beningo et al, 2004, Cukierman, 2001). One can reconstitute natural conditions by using different materials that can support 3-D cell growth, for example biodegradable polymers (Dhiman et al, 2004), hydrogels (Fisher et al, 2004, Park et al, 2005) etc. This cellular model was highly convenient for the selection of aptamers able to bind target cells in 3-D growth. The important feature in such a model is that the cells are capable to postconfluent growth, organizing high density multilayers with non-attached aggregates, mimicking condition of solid tumours.

Ten rounds of SELEX under gradually increasing selection pressure causes the generation a restricted number of aptamers, which bound target cells with different affinities. Some of these were highly abundant, most could be classified within a few principal families according to sequence and structure similarities, and few were found at a single example.

The Inventors addressed the effect on migration with a robust and quick wound healing assay and were able to identify modulators of cell migration using this in combination with the modified SELEX method. Of the total set of aptamers identified, only a few aptamers were shown to retard cell migration into wounds without changing the cell proliferation rate. Interestingly, the members of two large families containing highly represented strong binders did not show any effect on cells migration.

Two aptamers, E10 (SEQ ID NO: 4) and E37 (SEQ ID NO: 3) have been found to decrease the horizontal migration (wound healing) and vertical cell migration (transwell assay) of target cells, apparently resorting to different ways of action. Both are able to distinguish high metastatic cells from low metastatic ones. Both are highly specific and able to bind their target cells growing in 3D-dimentional aggregates. Aptamer E10 (SEQ ID NO: 4) binds HETSR-1 cells with high affinity (Kd 25nM). Fluorescent imaging showed that its binding sites are most probably at the cell surface. This is expected since cell-based SELEX generates usually aptamers against cell surface molecules, which are most probably responsible for not only cell migration but cell invasion as well.

The Inventors have shown that E10 (SEQ ID NO: 4) is even more active in terms of suppression of cell invasion. Aptamer E37 (SEQ ID NO: 3), although blocking migration more strongly than E10 (SEQ ID NO: 4), does not influence invasion and does not saturate its targets at concentrations up to 1µM. According to fluorescent images, this can be explained by its unexpected internalization into cells. E37 (SEQ ID NO: 3) can penetrate target cells in a non-saturable manner or saturation is achieved at a very high concentrations.

Cellular uptake of oligonucleotides is a known fact. It depends on cell division frequency, endocytic capacity of the cells etc. (Audouy and Hoekstra 2001; Simberg et al. 2004.). It was shown that naked DNA is able to get internalized in vitro by sperm (Lavitrano et al, 1992), epithelial (Zabner et al, 1997), endothelial (Nakamura et al, 1998) and muscle cells (Wolff et al, 1990). Traditional interpretation of the uptake of naked oligonucleotides involves endocytic mechanism, although it is considered that additional pathways exist (Wu-Pong 2000).

The mechanism underlying the antimigratory effects of aptamer E37 (SEQ ID NO: 3) involves at least initial binding to the cell surface and subsequent internalization.

It is possible that SELEX, based on frequently dividing cancer cells can lead to the generation of internalizing sequences.

### REFERENCES

1. Naumov, G., MacDonald, I., Weinmeister, P., Kerkvliet, N., Nadkami,K.V., Wilson, S.M., Morris, V.L., Groom, A.C., Chambers, A.F., 2002. Persistence of solitary mammary carcinoma cells in a secondary site: a possible contributor to dormancy. Cancer Res. 62, 2162- 2168.
2. Entschladen, F., Drell IV, T., Lang, K., Joseph, J., Zaenker, K., 2004. Cellcell migration, invasion, and metastasis: navigation by eurotransmitters. Lancet Oncol. 5, 254-258.
3. Douma, S., Van Laar, T., Zevenhoven, J., Meuwissen, R., Van Garderen, E., Peeper, D.S., 2004. Suppression of anoikis and induction of metastasis by the neurotrophic receptor TrkB. Nature 430, 1034- 1039.
4. Giese, A., Bjerkvig, R., Berens, M.E., Westphal, M., 2003. Cost of migration: invasion of malignant gliomas and implications for treatment. J. Clin. Oncol. 21, 1624- 1636.
5. Haga, A., Funasaka, T., Niinaka, Y., Raz, A., Nagase, H., 2003. Autocrine motility factor signaling induces tumor apoptotic resistance by regulations Apaf-1 and Caspase-9 apoptosome expression. Int. J. Cancer 107, 707-714.
6. Jayasena, S.D. Aptamers: an emerging class of molecules that rival antibodies in diagnostics. Clin.Chem. 9, 1628-1650 (1999).
7. Eyetech Study Group. Preclinical and phase IA clinical evaluation of an anti-VEGF pegylated aptamer (EYE001) for the treatment of exudative age-related macular degeneration. Retina 2002;22:143-52.
8. Hicke BJ, Stephens AW, Gould T, Chang YF, Lynott CK, Heil J, Borkowski S, Hilger CS, Cook G, Warren S, Schmidt PG. Tumor targeting by an aptamer. J Nucl Med. 2006 Apr;47(4):668-78.
9. Bates PJ, Kahlon JB, Thomas SD, Trent JO, Miller DM. Antiproliferative activity of G-rich oligonucleotides correlates with protein binding. J Biol Chem 1999;274:26369-77.
10. Systematic evolution of ligands by exponential enrichment: RNA ligands to bacteriophage T4 DNA polymerase C Tuerk and L Gold Science 3 August 1990 249: 505-510.
11. Ellington AD, Szostak JW. In vitro selection of RNA molecules that bind specific ligands. Nature. 1990 Aug 30;346(6287):818-22.
12. K.N. Morris, et al., High affinity ligands from in vitro selection: complex targets, Proc. Natl. Acad. Sci. USA 95 (6) (1998) 2902-2907.
13. M. Blank, et al., Systematic evolution of a DNA aptamer binding to rat brain tumor microvessels. Selective targeting of endothelial regulatory protein pigpen, J. Biol. Chem. 276 (19) (2001) 16464-16468.
14. D.A. Daniels, et al., A tenascin-C aptamer identified by tumor cell SELEX: systematic evolution of ligands by exponential enrichment, Proc. Natl. Acad. Sci. USA 100 (26) (2003) 15416-15421.
15. Cerchia L, Duconge F, Pestourie C, Boulay J, Aissouni Y, Gombert K, Tavitian B, de Franciscis V, Libri D. Neutralizing aptamers from whole-cell SELEX inhibit the RET receptor tyrosine kinase. PLoS Biol. 2005 Apr;3(4):e123. Epub 2005 Mar 22.
16. Wang C, Zhang M, Yang G, Zhang D, Ding H, et al. (2003) Single-stranded DNA aptamers that bind differentiated but not parental cells: Subtractive systematic evolution of ligands by exponential enrichment. J Biotechnol 102: 15-22.
17. Blank M, Weinschenk T, Priemer M, Schluesener H (2001) Systematic evolution of a DNA aptamer binding to rat brain tumor microvessels. Selective targeting of endothelial regulatory protein pigpen. J Biol Chem 276: 16464-16468.
18. Deichman GI, Topol LZ, Kluchareva TE, Matveeva VA, Zakamaldina TA, Uvarova EN, Tatosyan AG. Clustering of discrete cell properties essential for tumorigenicity and metastasis. III. Dissociation of the properties in N-ras-transfected RSV-SR-transformed cells. Int J Cancer. 1992 Jul 30;51(6):903-8.
19. Deichman GI, Kashleva HA, Kluchareva TE, Matveeva VA. Clustering of discrete cell properties essential for tumorigenicity and metastasis. II. Studies of Syrian hamster embryo fibroblasts transformed by Rous sarcoma virus. Int J Cancer. 1989 Nov 15;44(5):908-10.
20. Bartel DP, Szostak JW (1993) Isolation of new ribozymes from a large pool of random sequences. Science 261: 1411-1418.
21. Tatosyan A, Yatsula B, Shtutman M, Moinova E, Kaverina I, Musatkina E, Leskov K, Mizenina O, Zueva E, Calothy G, Dezelee P. Two novel variants of the v-src oncogene isolated from low and high metastatic RSV-transformed hamster cells. Virology. 1996 Feb 15;216(2):347-56.
22. Isachenko N, Dyakova N, Aushev V, Chepurnych T, Gurova K, Tatosyan A.High expression of shMDG1 gene is associated with low metastatic potential of tumor cells. Oncogene. 2006 Jan 12;25(2):317-22.
23. Mizenina O, Musatkina E, Yanushevich Y, Rodina A, Krasilnikov M, de Gunzburg J, Camonis JH, Tavitian A, Tatosyan A. A novel group IIA phospholipase A2 interacts with v-Src oncoprotein from RSV-transformed hamster cells. J Biol Chem. 2001 Sep 7;276(36):34006-12. Epub 2001 Jun 26.
24. Sawyer, T.K., 2004. Cancer metastasis therapeutic targets and drug discovery: emerging small-molecule protein kinase inhibitors. Expert. Opin. Invest. Drugs 13, 1- 19.
25. Tucker, G.C., 2003. Alpha v integrin inhibitors and cancer therapy. Curr. Opin. Invest. Drugs 4, 722- 731.
26. Zucker, S., Cao, J., Chen, W., 2000. Critical appraisal of the use of matrix metalloproteinase inhibitors in cancer treatment. Oncogene 19, 6642- 6650.
27. Overall, C., Lopez-Otin, C., 2002. Strategies for MMP inhibition in cancer: innovations for the post-trial era. Nat. Rev., Cancer 2, 657-672.
28. Coussens, L., Fingleton, B., Matrisian, L., 2002. Matrix metalloproteinase inhibitors and cancer: trials and tribulations. Science 295, 2387-2392.
29. Friedl, P., Wolf, K., 2003. Cell -cell invasion and migration: diversity and escape mechanisms. Nature 3, 362-374.
30. K.L. Schmeichel, M.J. Bissell, Modeling tissue-specific signaling and organ function in three dimensions, J. Cell Sci. 116 (2003) 2377-2388.
31. K.A. Beningo, M. Dembo, Y.L. Wang, Responses of fibroblasts to anchorage of dorsal extracellular matrix receptors, Proc. Natl. Acad. Sci. USA 101 (2004) 18024-18029.
32. E. Cukierman, R. Pankov, D.R. Stevens, K.M. Yamada, Taking cellmatrix adhesions to the third dimension, Science 294 (2001) 1708-1712.
33. Dhiman HK, Ray AR, Panda AK. Characterization and evaluation of chitosan matrix for in vitro growth of MCF-7 breast cancer cell lines. Biomaterials. 2004 Sep;25(21):5147-54. PMID: 15109838 [PubMed - indexed for MEDLINE].
34. J.P. Fisher, S. Jo, A.G. Mikos, A.H. Reddi, Thermoreversible hydrogel scaffolds for articular cartilage engineering, J. Biomed. Mater. Res. A 71 (2004) 268-274.
35. Y. Park, M. Sugimoto, A. Watrin, M. Chiquet, E.B. Hunziker, BMP-2 induces the expression of chondrocyte-specific genes in bovine synovium-derived progenitor cells cultured in three-dimensional alginate hydrogel, Osteoarthritis Cartilage 13 (2005) 527-536.
36. Audouy S, Hoekstra D. Cationic lipid-mediated transfection in vitro and in vivo (review). Mol Membr Biol. 2001 Apr-Jun;18(2):129-43. Review.
37. Audouy S, Hoekstra D. Cationic lipid-mediated transfection in vitro and in vivo (review). Mol Membr Biol. 2001 Apr-Jun;18(2):129-43. Review. Audouy and Hoekstra 2001; Simberg et al. 2004.
38. M. Lavitrano, D. French, M. Zani, L. Frati, C. Spadafora, The interaction between exogenous DNA and sperm cells, Mol. Reprod. Dev. 31 (1992) 161-169.
39. J. Zabner, S.H. Cheng, D. Meeker, J. Launspach, R. Balfour, M.A. Perricone, J.E. Morris, J. Marshall, A. Fasbender, A.E. Smitha, M.J. Welsh, Comparison of DNA-lipid complexes and DNA alone for gene transfer to cystic fibrosis airway epithelia in vivo, J. Clin. Invest. 15 (1997) 1529-1537.
40. M. Nakamura, P. Davila-Zavala, H. Tokuda, Y. Takakura, M. Hashida, Uptake and gene expression of naked plasmid DNA in cltured brain microvessel endothelial cells, Biochem. Biophys. Res. Commun. 245 (1998) 235-239.
41. J.A. Wolff, R.W. Malone, P. Williams, P.W. Chong, G. Acsadi, A. Jani, P.L. Felgner, Direct gene transfer into mouse muscle in vivo, Science 247 (1990) 1465-1468.
42. Wu-Pong S. Alternative interpretations of the oligonucleotide transport literature: insights from nature. Adv Drug Deliv Rev. 2000 Oct 31;44(1):59-70. Review.
43. Ireson CR, Kelland LR. Discovery and development of anti-cancer aptamers. Mol Cancer Ther. 2006 Dec;5(12):2957-62. Review.
44. D.H. Mathews et al., J. Mol. Biol., 1999, 288, 911-940.

## Claims

1. A method for identifying aptamers specific for at least one metastatic cancer cell marker, using a mixture of nucleic acids, wherein said method comprises at least the following steps:
(a) bringing a mixture of nucleic acids which form a combinatorial library into contact with cells not expressing said at least one metastatic cancer cell marker (C_{N} Cells), said C_{N} cells being the same cell type as metastatic cells expressing said at least one metastatic cancer cell marker (C_{M} cells);
(b) recovering a first subset S 1 of nucleic acids which do not bind to the C_{N} cells, in step (a);
(c) bringing the first subset S1 into contact with the C_{M} cells;
(d) recovering the nucleic acids which exhibit a high affinity with respect to the cells expressing said at least one metastatic cancer cell marker expressed by said C_{M} cells;
(e) amplifying said nucleic acids with high affinity for the cells expressing said at least one metastatic cancer cell marker, so as to obtain a mixture of nucleic acids, enriched in nucleic acids having a high affinity for said C_{M} cells, and
(f) identifying aptamers specific for the cells expressing said at least one metastatic cancer cell marker, from the mixture obtained in (e).

2. The method as claimed in claim 1, **characterized in that** steps (a) to (e) are repeated using the mixtures enriched in aptamers from the preceding cycle, until at least one aptamer is obtained, the affinity of which, defined by its dissociation constant (Kd), can be measured and is suitable for pharmaceutical use.

3. The method as claimed in claim 2, **characterized in that** steps (a) to (f) are repeated between at least 10 times and 15 times.

4. The method as claimed in claim 1, 2 or 3, **characterized in that** the starting nucleic acid combinatorial library contains at least 10⁹ nucleic acids, preferably between 10¹² and 10¹⁶ nucleic acids, and advantageously consists of nucleic acids comprising random sequences comprising, respectively at their 5' and 3' ends, fixed sequences for PCR amplification, wherein preferably said fixed sequences for PCR amplification are selected from the group consisting of SEQ ID NO: and SEQ ID NO:2 or a fragment of at least 8 nucleotides of these sequences.

5. The method as claimed in any one of claims 1 to 4, **characterized in that** said random sequences each contain between 10 and 1000 nucleotides, preferably 40 nucleotides, and comprise deoxyribonucleic acid, ribonucleic acid or modified nucleic acids.

6. The method as claimed in any one of claims 1 to 5, **characterized in that** the identification of the aptamers specific for the C_{M} cells according to step (f) comprises an evaluation of the biological activity of said aptamers on said C_{M} cells.

7. The method as claimed in claim 6, **characterized in that** said biological activities which are evaluated is selected from the following:
(a) inhibition or activation of horizontal cell migration,
(b) inhibition or activation of vertical cell migration,
(c) inhibition or activation of cell invasion.

8. An aptamer, **characterized in that** it is specific for cells expressing at least one metastatic cancer cell marker, and can be identified by means of the method for identifying aptamers as claimed in any one of claims 1 to 7.

9. The aptamer as claimed in claim 8, **characterized in that** it recognises at least one metastatic cancer cell marker, and in particular at least one metastatic cancer cell marker induced by infection of said cell by an oncovirus.

10. The aptamer as claimed in claim 8 or claim 9, **characterized in that** said oncovirus is Rous sarcoma virus.

11. The aptamer as claimed in claim 8, 9, 10 or 11, **characterized in that** it can be identified by means of the method comprising:
(a) bringing a mixture of nucleic acids into contact with C_{N} cells infected with an oncovirus and not expressing at least one metastatic cancer cell marker form,
(b) recovering a first subset S1 of nucleic acids which do not bind to said C_{N} cells, in step (a),
(c) bringing said first subset S1 into contact with C_{M} cells infected with an oncovirus and expressing said at least one metastatic cancer cell marker,
(d) recovering the nucleic acids which exhibit a high affinity with respect to the cells expressing said at least one metastatic cancer cell marker expressed by said C_{M} cells, after dissociation of the cell-nucleic acid complexes;
(e) amplifying said nucleic acids with high affinity for the cells expressing said at least one metastatic cancer cell marker, so as to obtain a mixture of nucleic acids, enriched in nucleic acids having a high affinity for said C_{M} cells, and
(f) repeating steps (a)-(e), until at least one aptamer is obtained, the affinity of which for the C_{M} cells, defined by its dissociation constant (Kd), is measurable and suitable for a pharmacological activity, and
(g) identifying the aptamers specific for the cells expressing at least one metastatic cancer cell marker, selected from the mixture obtained in (f).

12. An aptamer, **characterized in that** it can be obtained by means of a method of identification as defined in claims 1 to 7, and **in that** it is selected from aptamers of formula (I):
R₁-R-R₂ (I),
in which:
R₁ represents 5' AGATTGCACTTACTCGAA 3' (SEQ ID NO: 1) or a fragment of 1 to 18 nucleotides of said SEQ ID NO: 1;
R₂ represents 5' GGAATGAATAAGCTGGTATCTCCC 3' (SEQ ID NO:2) or a fragment of 1 to 24 nucleotides of said SEQ ID NO:2, and
R represents a random sequence of 10 to 1000 nucleotides, preferably 40 nucleotides.

13. The aptamer as claimed in anyone of claims 8 to 12, **characterized in that** the ribose of each purine have a hydroxyl group or a fluorine atom on the carbon in the 2'-position, while the ribose of each pyrimidine have a fluorine atom on the carbon in the 2'-position.

14. The aptamer as claimed in any one of claims 8 to 13, **characterized in that** it is selected from the sequences SEQ ID NO: 3-36.

15. A reagent for diagnosing a tumor, **characterized in that** it consists of an aptamer as claimed in any one of claims 8 to 14.

16. A medicament, **characterized in that** it comprises an aptamer selected from the group: SEQ ID NO: 3, SEQ ID NO: 4, which has the ability to inhibit metastatic cell migration.

17. A pharmaceutical composition, **characterized in that** it comprises:
an aptamer selected from the group: SEQ ID NO: 3, SEQ ID NO: 4, which has the ability to inhibit metastatic cell migration, and
at least one pharmaceutically acceptable vehicle.

18. The use of an aptamer as claimed in any one of claims 8 to 14, for screening products which interact with said at least one metastatic cancer cell marker and which affect the migration or invasiveness of a cell expressing said at least one metastatic cancer cell marker.

19. A method for screening substances which interact with at least one metastatic cancer cell marker or targets forming a complex with said at least one metastatic cancer cell marker, wherein said method is **characterized in that** it comprises:
bringing cells expressing said at least one metastatic cancer cell marker into contact with the substance to be tested,
adding, under suitable conditions, an aptamer as claimed in any one of claims 8 to 14, before, at the same time as or after the substance to be tested,
evaluating the competitive binding between said aptamer and said substance to be tested.

20. The method as claimed in claim 19, **characterized in that,** after identification of said interacting substance which binds competitively with the aptamer to the cells expressing said at least one metastatic cancer cell marker, the effect of said substance on the biological activity of said cells can be evaluated in order to find substances which inhibit or activate said biological activities of the cells exhibiting said at least one metastatic cancer cell marker.

21. A method for identifying at least one molecular target of an aptamer according to any one of claims 8 to 14, comprising at least the steps of:
(a) bringing at least one of said aptamer into contact with cells expressing at least one metastatic cancer cell marker expressed by C_{M} cells;
(b) recovering said at least one aptamer whilst still in complex with said at least one molecular target of said C_{M} cells; and
(c) identifying said at least one molecular target.

22. A method for identifying at least one molecular target of an aptamer according to any one of claims 8 to 14, comprising at least the steps of:
(a) bringing at least one of said aptamer into contact with cells expressing at least one metastatic cancer cell marker expressed by C_{M} cells;
(b) recovering the total mRNA content of said cells;
(c) comparing by microarray analysis the mRNA sample of step (b) to that of a control mRNA sample; and
(d) identifying said at least one molecular target.

23. A method for identifying at least one molecular target of an aptamer according to any one of claims 8 to 14, comprising at least the steps of:
(a) bringing at least one of said aptamer into contact with cells expressing at least one metastatic cancer cell marker expressed by C_{M} cells;
(b) recovering the total protein content of said cells;
(c) comparing by microarray analysis the protein sample of step (b) to that of a control protein sample; and
(d) identifying said at least one molecular target.
